(19) **Europäisches Patentamt**

**European Patent Office**

**Office européen des brevets**

(11) Veröffentlichungsnummer: **0 676 468 A2**

(12) # EUROPÄISCHE PATENTANMELDUNG

(21) Anmeldenummer: **95103785.2**

(22) Anmeldetag: **15.03.95**

(51) Int. Cl.6: **C12N 15/12**, C07K 14/725,
C07K 16/28, A61K 38/17,
A61K 39/395, C12N 5/10

Der Anmelder hat nachträglich ein Sequenzprotokoll eingereicht und erklärt, dass dieses keine neuen Angaben enthält.

(30) Priorität: **16.03.94 DE 4408999**

(43) Veröffentlichungstag der Anmeldung:
**11.10.95 Patentblatt 95/41**

(84) Benannte Vertragsstaaten:
**AT BE CH DE DK ES FR GB GR IE IT LI LU MC NL PT SE**

(71) Anmelder: **B. BRAUN MELSUNGEN AG**
**Carl-Braun-Strasse 1**
**D-34212 Melsungen (DE)**

(72) Erfinder: **Ziegler, Annette G., Dr.**
**Hedwigstrasse 11**
**D-80636 München (DE)**
Erfinder: **Schendel, Dolores, Prof. Dr.**
**Hans Sachs Strasse 12**
**D-41065 München (DE)**
Erfinder: **Steinle, Alexander**
**Dachauer Strasse 163**
**D-80992 München (DE)**
Erfinder: **Durinovic-Bello, Ivana, Dr.**
**Tatzelwurmweg 7**
**D-82031 Grünwald (DE)**

(74) Vertreter: **Weiss, Wolfgang, Dr. Dipl.-Chem. et al**
**Patentanwälte**
**Weickmann & Partner,**
**Kopernikusstrasse 9**
**D-81679 München (DE)**

(54) **Humane T-Zellrezeptoren zur diagnostischen sowie therapeutischen Verwendung bei autoimmunem Diabetes mellitus.**

(57) Die Erfindung betrifft neue Nukleinsäure- und Aminosäuresequenzen des humanen T-Zellrezeptors und deren Verwendung für die Diagnostik und Therapie des autoimmunen Typ I Diabetes mellitus.

EP 0 676 468 A2

Die Erfindung betrifft neue Nukleinsäure- und Aminosäuresequenzen des humanen T-Zellrezeptors und deren Verwendung für die Diagnostik und Therapie des autoimmunen Typ I Diabetes mellitus.

Der Typ I Diabetes mellitus ist nach heutigen Erkenntnissen eine Autoimmunerkrankung, die durch eine gezielte Zerstörung der Insulin-produzierenden $\beta$-Zellen der Langerhans-Inseln im Pankreas verursacht wird. Es gibt Hinweise, daß diese Zerstörung durch autoreaktive T-Lymphozyten vermittelt wird, die mit spezifischen "Betazellen-Selbstpeptiden" (Autoantigenen) reagieren.

Die Bindung der T-Zellen an Autoantigene wird über den T-Zellrezeptor vermittelt. Das Bindungsrepertoire von T-Zellrezeptoren wird durch positive und negative Selektion über eine Reaktion mit Selbst-Peptiden bestimmt, die an Moleküle des Major Histocompatibility Complex (MHC) gebunden sind (Davies und Bjorkmann (1988), Nature 334: 395). Bei humanen Autoimmunkrankheiten, wie etwa der Multiplen Sklerose, der rheumatoiden Arthritis und autoimmunen Schilddrüsenkrankheiten wurde ein definiertes TCR-Repertoire in Lymphozyten gefunden, welche die betroffenen Organe infiltrieren (Oksenberg et al. (1990), Nature 345: 344; Paliard et al. (1991), Science 253: 325; Davies et al. (1991), N. Engl. J. Med. 325: 238).

Beim autoimmunen Typ I Diabetes mellitus gibt es nur wenige Untersuchungen über das TCR-Repertoire in Insel-infiltrierenden Lymphozyten, da Pankreasgewebe nur sehr schwer erhältlich ist. Yamagata et al. ((1993), Autoimmunity 15: 52) beschreiben eine dominante Verwendung von TCR-V$\alpha$4- und V$\alpha$6-Gensegmenten in Pankreasbiopsien bei Typ I Diabetes-Patienten. Trucco et al. ((1993), Diabetes 42: Suppl. 1 4a) beschreiben eine prominente Expression des TCR-V$\beta$7-Gensegments in T-Zellen, die aus den Langerhans-Inseln eines an Typ I Diabetes verstorbenen Kindes isoliert wurden. Weitere Untersuchungen des TCR-V$\beta$-Repertoires in peripheren mononukleären Blutzellen (PBMC) von Diabetes-Patienten im Frühstadium (Kontiainen et al. (1991), Clin. Exp. Immunol. 83: 347; Wong et al. (1993), Autoimmunity 15: 52) oder bei Zwillingen (Malhotra et al. (1992), Immunol. 149: 1802) zeigten keine Assoziierung mit einer der untersuchten V$\beta$-Gen-Familien.

Ein Grund für das bisherige Scheitern, definierte Muster der TCR-Verwendung in peripherem Blut von frisch diagnostizierten Typ I Diabetes-Patienten zu identifizieren, kann darin bestehen, daß die T-Zellen einer Reihe von verschiedenen Autoantigenen ausgesetzt sind, die während des Krankheitsverlaufs auftreten.

Eine Aufgabe der vorliegenden Erfindung bestand somit darin, Nukleinsäure- und Aminosäuresequenzen von T-Zellrezeptoren bereitzustellen, die mit Autoantigenen aus $\beta$-Zellen der Langerhans-Inseln spezifisch reagieren.

Erfindungsgemäß wurde diese Aufgabe gelöst durch Isolierung von T-Zellklonen aus einem Patienten mit Typ I Diabetes, die spezifisch mit Betazell-Membranfragmenten reagieren, und Bestimmung der Nukleinsäure- und Aminosäuresequenz der T-Zellrezeptormoleküle in diesen Klonen. Insbesondere wurden CDR-3-Regionen der $\alpha$- und $\beta$-Ketten der T-Zellrezeptoren bestimmt, bei der es sich vermutlich genau um die Region im T-Zellrezeptor handelt, die mit dem erkannten Antigen interagiert.

Ein Gegenstand der vorliegenden Erfindung ist somit eine Nukleinsäure, die für einen Abschnitt aus der CDR3-Region einer Kette eines humanen T-Zellrezeptors codiert, umfassend:

(a) eine der in SEQ ID NO. 1 - 6 dargestellten Nukleinsäuresequenzen,

(b) eine Nukleinsäuresequenz, die einer der Sequenzen aus (a) im Rahmen der Degeneration des genetischen Codes entspricht, oder

(c) eine Nukleinsäuresequenz, die eine Homologie von mindestens 80 % zu einer Nukleinsäuresequenz aus (a) oder/und (b) aufweist.

Die in SEQ ID NO. 1 - 6 gezeigten Nukleinsäure- und Aminosäuresequenzen sind Bereiche aus den CDR3-Regionen der T-Zellrezeptoren aus den gefundenen T-Zellklonen. SEQ ID NO. 1 bzw. SEQ ID NO. 4 stammen aus der $\alpha$- bzw. $\beta$-Kette des Klons K2.12. SEQ ID NO. 2 bzw. SEQ ID NO. 5 stammen aus der $\alpha$- bzw. $\beta$-Kette des Klons K2.16. SEQ ID NO. 3 bzw. SEQ ID NO. 6 stammen aus der $\alpha$- bzw. $\beta$-Kette des Klons K2.4.

Neben den in SEQ ID NO. 1 - 6 dargestellten Nukleinsäuresequenzen oder diesen Sequenzen im Rahmen der Degeneration des genetischen Codes entsprechenden Nukleinsäuresequenzen umfaßt die vorliegende Erfindung auch solche Nukleinsäuresequenzen, die eine Homologie von mindestens 80 %, vorzugsweise von mindestens 90 % zu einer der oben genannten Sequenzen aufweisen.

Vorzugsweise enthält die erfindungsgemäße Nukleinsäure noch ein oder mehrere weitere Nukleotide und codiert für eine vollständige CDR3-Region einer Kette eines humanen T-Zellrezeptors. Besonders bevorzugt enthält sie

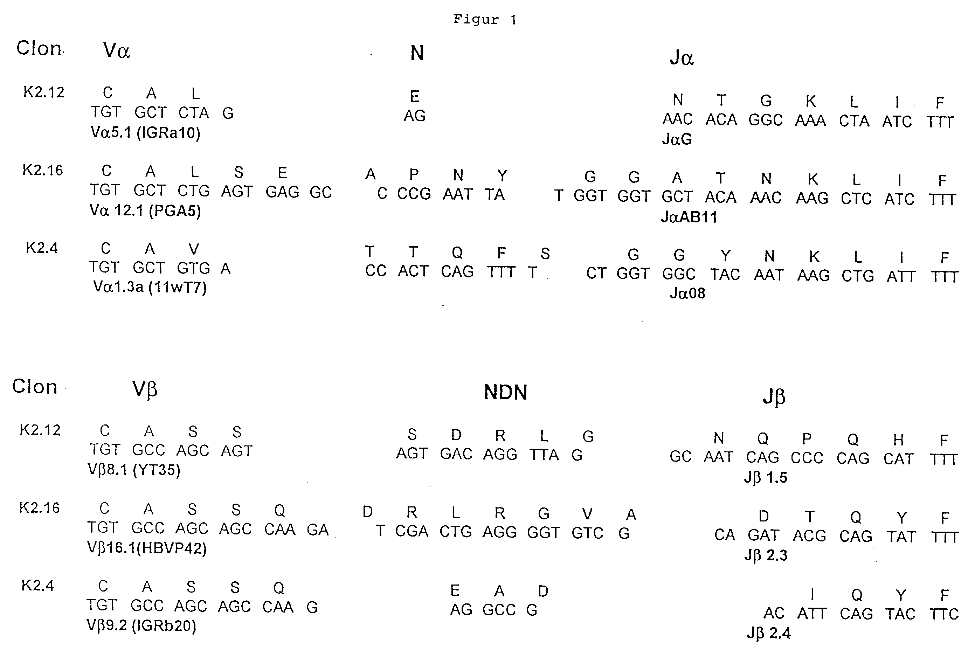

(a) eine der in Fig. 1 dargestellten Nukleinsäuresequenzen, in der die CDR3-Regionen der $\alpha$- und $\beta$-Ketten der gefundenen T-Zellklone K2.12, K2.16 und K2.4 vollständig gezeigt sind,

(b) eine Nukleinsäuresequenz, die einer der Sequenzen aus (a) im Rahmen der Degeneration des genetischen Codes entspricht, oder

(c) eine Nukleinsäuresequenz, die eine Homologie von mindestens 80 % zu einer Nukleinsäuresequenz aus (a) oder/und (b) aufweist.

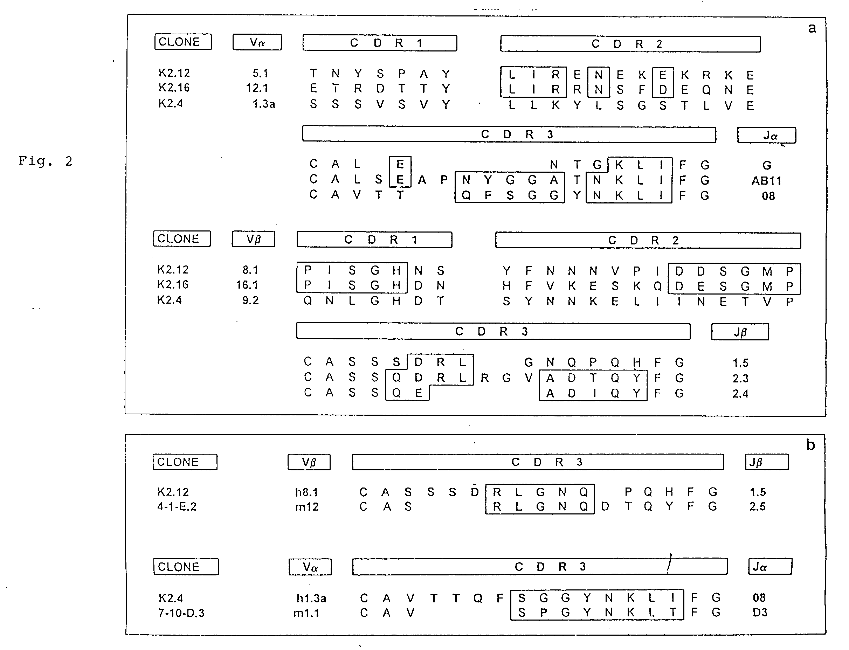

Ein weiterer Gegenstand der vorliegenden Erfindung ist eine Nukleinsäure, die für eine Kette eines humanen T-Zellrezeptors oder eines funktionellen Derivats davon codiert und die dadurch gekennzeichnet ist, daß ihre CDR3-Region eine Nukleinsäure wie oben definiert enthält. Vorzugsweise enthält diese Nukleinsäure weiterhin als CDR1- und CDR2-Regionen Nukleinsäuresequenzen, welche für die in Fig. 2 gezeigten Aminosäuresequenzen codieren. Diese Nukleinsäuresequenzen können aus den in Fig. 2 gezeigten Aminosäuresequenzen und dem universellen genetischen Code auf einfache Weise ermittelt werden.

Unter dem Begriff "funktionelles Derivat einer Kette eines humanen T-Zellrezeptors" im Sinne der vorliegenden Erfindung ist ein Polypeptid zu verstehen, das zusammen mit der komplementären Kette des humanen T-Zellrezeptors (oder einem Derivat einer solchen Kette) ein T-Zellrezeptor-Derivat bilden kann, das eine äquivalente Erkennungsspezifität für einen von einem MHC-Molekül präsentierten Peptidliganden wie der nicht-derivatisierte T-Zellrezeptor besitzt. Vorzugsweise weist ein derartiges T-Zellrezeptorderivat eine Bindungskonstante von mindestens $10^{-4}$ l/mol, vorzugsweise $10^{-4}$ bis $10^{-5}$ l/mol für den präsentierten Peptidliganden auf.

Die Herstellung funktioneller Derivate von Ketten eines humanen T-Zellrezeptors kann beispielsweise durch Deletion, Substitution oder/und Insertion von Abschnitten des für das jeweilige Polypeptid codierenden Gens durch rekombinante DNA-Techniken erfolgen. Die Herstellung von rekombinanten T-Zellrezeptorketten ist beispielsweise bei Blank et al. (1993), Eur. J. Immunol. 23, 3057-3065; Lin et al. (1990), Science 249: 677; Gregoire et al. (1991); Proc. Natl. Acad. Sci. USA 88: 8077; Kappes und Tonegawa (1991), Proc. Natl. Acad. Sci. USA 88: 10619 und Ward (1991), Scand. J. Immunol. 34: 215 beschrieben. Auf diese Literaturstellen wird hiermit ausdrücklich Bezug genommen.

Wenn die erfindungsgemäße Nukleinsäure für eine $\alpha$-Kette eines humanen T-Zellrezeptors oder eines funktionellen Derivats davon codiert, enthält sie vorzugsweise ein exprimiertes V-Gensegment aus den Genfamilien V$\alpha$1, V$\alpha$5 oder V$\alpha$12, insbesondere V$\alpha$1.3a, V$\alpha$5.1 oder V$\alpha$12.1. Weiterhin enthalten die erfindungsgemäßen Nukleinsäuren vorzugsweise exprimierte J$\alpha$G, J$\alpha$AB11 oder J$\alpha$08-Gensegmente.

Wenn die erfindungsgemäße Nukleinsäure für eine $\beta$-Kette eines humanen T-Zellrezeptors oder eines funktionellen Derivats davon codiert, enthält sie vorzugsweise ein exprimiertes V-Gensegment aus den Genfamilien V$\beta$8, V$\beta$9 oder V$\beta$16, insbesondere V$\beta$8.1, V$\beta$9.2 oder V$\beta$16.1. Weiterhin ist es bevorzugt, daß die erfindungsgemäße Nukleinsäure ein exprimiertes J$\beta$1.5, J$\beta$2.4 oder J$\beta$2.3 Gensegment enthält.

Die $\alpha$-Kette des T-Zellrezeptors aus dem Klon K2.12 wurde aus V$\alpha$5.1 und J$\alpha$G, die des Klons K2.16 aus V$\alpha$12.1 und J$\alpha$AB11 und die des Klons K2.4 aus V$\alpha$1.3a und J$\alpha$08-Gensegmenten kombiniert. Die $\beta$-Kette des T-Zellklons K2.12 wurde aus V$\beta$8.1 und J$\beta$1.5, die des Klons 2.16 aus V$\beta$16.1 und J$\beta$2.3 und die des Klons K2.4 aus V$\beta$9.2 und J$\beta$2.4 Gensegmenten kombiniert. Angaben zu den Nuklein- bzw. Aminosäuresequenzen für V- und J-Gensegmente finden sich für V$\alpha$1.3 bei Obata und Kashiwagi, in Tsuji et al. (Hrsg.), HLA 1991, Bd. 1, S. 865, Oxford University Press (1992); für V$\alpha$5.1 und J$\alpha$08 bei Roman-Roman et al. (1991), Eur. J. Immunol. 21: 927; für V$\alpha$12.1 bei Sim et al. (1984), Nature 312: 771; für J$\alpha$G bei Yoshikai et al. (1986), J. Exp. Med. 164: 90; für J$\alpha$AB11 bei Klein et al. (1987), Proc. Natl. Acad. Sci. USA 84: 6884; für V$\beta$8.1 bei Yanagi et al. (1984), Nature 308: 145; für V$\beta$9.2 bei Ferradini et al. (1991), Eur. J. Immunol. 21: 935; für V$\beta$16.1 bei Kimura et al. (1986), J. Exp. Med. 164: 739 und für J$\beta$ bei Toyonaga et al. (1985), Proc. Natl. Acad. Sci. USA 82: 8624. Auf diese Literaturstellen wird ausdrücklich Bezug genommen.

Ein weiterer Gegenstand der vorliegenden Erfindung ist ein Vektor, der mindestens eine Kopie einer erfindungsgemäßen Nukleinsäure enthält. Der Vektor kann ein prokaryontischer Vektor oder ein eukaryontischer Vektor sein. Beispiele für prokaryontische Vektoren sind Plasmide, Cosmide, Bakteriophagenvektoren (Bacteriophage lambda oder einzelsträngige filamentöse Bakteriophagen, z.B. M13). Diese Vektoren sind in Sambrook et al., Molecular Cloning. A Laboratory Manual, 2nd Edition (1989), Cold Spring Harbor Laboratory Press, in den Kapiteln 1 bis 4 ausführlich beschrieben. Vorzugsweise ist der prokaryontische Vektor ein Plasmid.

Andererseits kann der Vektor auch ein eukaryontischer Vektor sein, z.B. ein Hefevektor, ein Pflanzenvektor (Baculovirus) oder ein Säugervektor (SV40, Rinder-Papillomavirus, Epstein-Barr-Virus). Beispiele für eukaryontische Vektoren sind in Sambrook et al., supra, Kapitel 16 und Winnacker, Gene und Klone, Eine Einführung in die Gentechnologie (1985), VCH Verlagsgesellschaft, insbesondere in dem Kapiteln 5, 8 und 10 beschrieben.

Ein weiterer Gegenstand der vorliegenden Erfindung ist eine Zelle, die mit einer erfindungsgemäßen Nukleinsäure oder mit einem erfindungsgemäßen Vektor transformiert ist. Die Zelle kann eine prokaryontische Zelle (z.B. eine gram-negative Bakterienzelle, insbesondere E.coli) oder eine eukaryontische Zelle (z.B. eine Hefe-, Pflanzen- oder Säugerzelle) sein. Beispiele für geeignete Zellen und Verfahren zum Einführen der erfindungsgemäßen Nukleinsäure in derartige Zellen finden sich in den obigen Literaturstellen.

Ein weiterer Gegenstand der vorliegenden Erfindung ist ein Polypeptid, das von einer erfindungsgemäßen Nukleinsäure codiert ist. Vorzugsweise ist das Polypeptid eine Kette eines humanen T-Zellrezeptors oder ein funktionelles Derivat.

In einem weiteren Aspekt betrifft die Erfindung ein Polypeptid, das eine Kette eines humanen T-Zellrezeptors oder ein funktionelles Derivat davon ist und als Abschnitt der CDR3-Region

(a) eine der in SEQ ID NO. 1 - 6 gezeigten Aminosäuresequenzen oder

(b) eine Aminosäuresequenz mit einer äquivalenten Erkennungsspezifität für die Peptidkomponente des T-Zellrezeptorliganden aufweist.

Vorzugsweise umfaßt das erfindungsgemäße Polypeptid eine vollständige CDR3-Region einer Kette eines humanen T-Zellrezeptors, die neben den in SEQ ID NO. 1 - 6 angegebenen Sequenzen zusätzlich weitere Aminosäuren und insbesondere eine der in Fig. 1 dargestellten vollständigen CDR3-Aminosäuresequenzen enthält. Besonders bevorzugt enthält das Polypeptid weiterhin eine der in Fig. 2 dargestellten CDR1 oder/und CDR2-Aminosäuresequenzen.

Die Erfindung betrifft neben Polypeptiden, welche die in SEQ ID NO. 1 - 6 gezeigten Aminosäuresequenzen aufweisen, auch noch solche Polypeptide, die eine Aminosäuresequenz mit einer äquivalenten Erkennungsspezifität für die Peptidkomponente des T-Zellrezeptorliganden aufweisen. Eine äquivalente Erkennungsspezifität liegt vor, wenn die Proliferation von T-Zellen, die den entsprechenden T-Zellrezeptor tragen, durch eine Betazell-Membranantigenpräparation, die z.B. aus Ratteninsulinomzellen, Ratteninselzellen, humanen Inselzellen oder NOD-Mausinselzellen erhältlich ist, stimulierbar ist.

Vorzugsweise besitzt ein derartiges Polypeptid als Abschnitt der CDR3-Region eine der in SEQ ID NO. 1 - 6 dargestellten Aminosäuresequenzen. Andererseits kann das Polypeptid als CDR3-Region auch eine aus den in SEQ ID NO. 1 - 6 dargestellten Aminosäuresequenzen durch einen oder mehrere Aminosäureaustausche abgeleitete Sequenz aufweisen. Diese Aminosäureaustausche sind vorzugsweise konservative Aminosäureaustausche. Beispiele für solche konservativen Aminosäureaustausche sind der Austausch einer hydrophoben Aminosäure gegen eine andere hydrophobe Aminosäure (z.B. Leu gegen Ile oder Ala), der Austausch einer sauren Aminosäure gegen eine andere saure Aminosäure (z.B. Glu gegen Asp), der Austausch einer basischen Aminosäure gegen eine andere basische Aminosäure (z.B. Arg gegen Lys) oder der Austausch einer polaren Aminosäure gegen eine andere polare Aminosäure (Asn gegen Gln oder Ser gegen Thr). Weitere Beispiele für konservative Aminosäureaustausche finden sich bei Dayhoff et al., in Atlas of Protein Sequence and Structure, Vol. 5, Suppl. 3 (ed. M.O. Dayhoff), p. 345, The National Biomedical Research Foundation, Washington, DC (1979).

Ein weiterer Gegenstand der vorliegenden Erfindung ist ein Polypeptid, das T-Zell-Rezeptoreigenschaften, d.h. insbesondere die Fähigkeit zur Erkennung und Bindung eines von einem MHC-Molekül präsentierten Peptidliganden mit einer Bindungskonstante von vorzugsweise mindestens $10^{-4}$ l/mol aufweist und aus zwei der vorstehend genannten erfindungsgemäßen Polypeptide als Untereinheiten aufgebaut ist, wobei eine Polypeptiduntereinheit eine $\alpha$-Kette eines humanen T-Zellrezeptors oder ein funktionelles Derivat davon ist und die andere Polypeptiduntereinheit eine $\beta$-Kette eines humanen T-Zellrezeptors oder ein funktionelles Derivat davon ist.

Die Herstellung eines solchen Polypeptids mit T-Zell-Rezeptoreigenschaften kann durch gemeinsame Expression der $\alpha$- und $\beta$-Kettenuntereinheiten in einer geeigneten Wirtszelle, z.B. einer Bakterien- oder einer Säugerzelle und anschließende Isolierung des Produkts aus der Zelle oder durch getrennte Expression der einzelnen Untereinheiten und Rekombination in vitro erfolgen.

Ein weiterer Gegenstand der vorliegenden Erfindung ist ein Antikörper gegen ein erfindungsgemäßes Polypeptid, der gegen eine für die Erkennung des Peptidliganden verantwortliche Region des Polypeptids gerichtet ist. Vorzugsweise ist dieser Antikörper ein monoklonaler Antikörper oder ein Fragment eines monoklonalen Antikörpers (z.B. ein Fab-, F(ab)$_2$-, Fab' oder F(ab')$_2$-Fragment). Vorzugsweise ist der Antikörper gegen eine CDR3-Region des Polypeptids oder einen Bereich davon gerichtet. Derartige Antikörper können nach an sich bekannten Methoden durch Immunisierung eines Versuchstiers mit einem Peptid oder Polypeptid, welches eine erfindungsgemäße CDR3-Region oder einen Abschnitt davon enthält, und Gewinnung der resultierenden Antikörper aus dem Versuchstier erhalten werden. Monoklonale Antikörper können durch Fusion einer Antikörper-produzierenden B-Zelle des Versuchstiers mit einer Leukämiezelle nach der Methode von Köhler und Milstein oder einer Weiterentwicklung davon erhalten werden. Spezifische Beispiele für die Herstellung solcher Antikörper finden sich bei Choi et al. (1991), Proc. Natl. Acad. Sci. USA 88: 8357 und Zumla et al. (1992), Hum. Immunol. 35: 141.

Ein weiterer Gegenstand der vorliegenden Erfindung ist eine T-Zelle, die einen erfindungsgemäßen T-Zellrezeptor enthält. Derartige T-Zellen können aus Patienten mit Typ I Diabetes isoliert werden. Hierzu können beispielsweise die peripheren mononukleären Blutzellen eines Patienten durch Stimulation mit Membranantigenen aus Ratteninsulinomzellen und anschließender Restimulation durch eine bestrahlte autologe Lymphoblastoidzellinie erzeugt werden.

Ein weiterer Gegenstand der vorliegenden Erfindung ist eine pharmazeutische Zusammensetzung, die als aktive Komponente eine erfindungsgemäße Nukleinsäure, ein erfindungsgemäßes Polypeptid, einen erfindungsgemäßen Antikörper oder eine erfindungsgemäße T-Zelle als Wirkstoff enthält. Diese pharmazeutische Zusammensetzung kann vorzugsweise zur Herstellung eines diagnostischen oder/und therapeutischen Mittels für Typ I Diabetes verwendet werden.

Der erfindungsgemäße Antikörper, der den Komplex von Autoantigen und T-Zellrezeptor erkennt, kann als spezifisches immunsupressives Mittel eingesetzt werden, z.B. als Impfstoff gegen autoreaktive T-Zellpopulationen in einem Typ I Diabetes-Patienten.

Weiterhin könnte das erfindungsgemäße Polypeptid oder die erfindungsgemäße T-Zelle zur Herstellung eines Immunogens verwendet werden, wodurch die Erzeugung von spezifischen Antikörpern gegen die entsprechende variable Region des T-Zellrezeptors im Empfänger bewirkt werden kann. Bei Verwendung von ganzen T-Zellen als Immunogen sollten diese attenuiert sein, d.h. nicht mehr zur Proliferation in der Lage sein. Diese Attenuierung erfolgt vorzugsweise durch Bestrahlung oder/und Hitzebehandlung. Beispiele für eine T-Zellvakzinierung sind etwa bei Cohen und Weiner (1988), Immunology Today 9: 332, Lider et al. (1988), Science 239: 181; Lohse et al. (1989), Science 244: 820 und Mor et al. (1990), J. Clin. Invest. 85: 1594 beschrieben. Auf diese Techniken wird ausdrücklich Bezug genommen.

Schließlich betrifft die Erfindung auch einen Komplex, der einen erfindungsgemäßen T-Zellrezeptor und ein Peptid-präsentierendes HLA-Molekül der Klasse DQw1 umfaßt.

Weiterhin wird die vorliegende Erfindung durch die folgenden Abbildungen und Sequenzprotokolle erläutert.

Es zeigen:

Fig. 1 die Nukleinsäure- und Aminosäuresequenzen der T-Zellrezeptoren aus den humanen T-Zellklonen K2.12, K2.16 und K2.4 in den Verbindungsregionen zwischen V-und J-Gensegmenten (N- bzw. NDN-Regionen),

Fig. 2a die Aminosäuresequenzen der T-Zellrezeptoren aus den humanen T-Zellklonen K2.12, K2.16 und K2.4 in den CDR1-, CDR2- und CDR3-Regionen,

Fig. 2b einen Vergleich der CDR3-Regionen der α-Kette des humanen T-Zellklons K2.4 und der β-Kette des humanen T-Zellklons K2.12 mit murinen T-Zellrezeptoren,

Fig. 3 die Variation der Expression von TCR-Vα- und Vβ-Genfamilien bei Stimulierung der Proliferation von T-Zellen aus einem Diabetes-Patienten mit Betazell-Membranantigenen,

SEQ ID NO. 1: die Nukleinsäure- und Aminosäuresequenz eines Abschnitts aus der CDR3-Region der α-Kette des TCR aus dem Klon K2.12,

SEQ ID NO. 2: die Nukleinsäure- und Aminosäuresequenz eines Abschnitts aus der CDR3-Region der α-Kette des TCR aus dem Klon K2.16,

SEQ ID NO. 3: die Nukleinsäure- und Aminosäuresequenz eines Abschnitts aus der CDR3-Region der α-Kette des TCR aus dem Klon K2.4,

SEQ ID NO. 4: die Nukleinsäure- und Aminosäuresequenz eines Abschnitts aus der CDR3-Region der β-Kette des TCR aus dem Klon K2.12,

SEQ ID NO. 5: die Nukleinsäure- und Aminosäuresequenz eines Abschnitts aus der CDR3-Region der β-Kette des TCR aus dem Klon K2.16 und

SEQ ID NO. 6: die Nukleinsäure- und Aminosäuresequenz eines Abschnitts aus der CDR3-Region der β-Kette des TCR aus dem Klon K2.4.

Beispiel 1

Herstellung von Membranantigenen

Membranantigene aus Ratteninsulinomzellen 5AHT2 (Beta-Membranantigen, BMA), Ratteninselzellen, Rattenmilz aus adulten Wistar-Ratten, NOD-Mausinselzellen, humanen Inselzellen, humanen CAKI-Zellen (ATCC HTB46) und SW579-Zellen (ATCC HTB107) wurden nach dem Verfahren von van Vliet ((1989), Eur. J. Immunol 19: 213) hergestellt. Die Zellinien oder Gewebehomogenisate wurden in Saccharosepuffer (0,32 M Saccharose, pH 7,4) bei 4 °C resuspendiert, aliquotiert und in einem Glasgefäß unter Verwendung eines Teflonstabes homogenisiert. Das Aufbrechen der Zellen wurde mikroskopisch überwacht. Die Suspension von Zellfragmenten wurde für 5 Minuten bei 900 g zentrifugiert. Der resultierende Überstand wurde für 30 Minuten bei 13000 g zur Pelletierung der Membranen erneut zentrifugiert. Diese Membranen wurden zweimal in Tris-HCl (50 mM, pH 7,4) mit wiederholter Zentrifugation für 20 Minuten bei 27000 g gewaschen. Das Pellet wurde in Tris-HCl resuspendiert und Aliquots wurden bei -70°C aufbewart. Der Proteingehalt wurde nach dem Verfahren von Lowry et al. ((1951), J. Biol. Chem. 193: 265) unter Verwendung von Rinderserumalbumin als Standard ermittelt.

Beispiel 2

Erzeugung von Antigen-spezifischen T-Zellen

Aus peripheren mononukleären Blutzellen eines Typ I Diabetes-Patienten, die in Kulturmedium unter Zusatz von 10 % Hitze-inaktiviertem Humanserum, 25 % T-Zellwachstumsfaktor (Lotze et al. (1980), J. Immunol. 124: 2927) und 40 $\mu$g/ml der in Beispiel 1 hergestellten BMA-Präparation stimuliert wurden, wurde eine T-Zellinie erhalten. Nach 24 h wurde neues Kulturmedium mit 40 U/ml rekombinantem IL-2 zugesetzt. Die T-Zellinie wurde wöchentlich durch Zugabe von bestrahlten (60 Gy) autologen Lymphoblastoidzellen (LCL) als Antigen-präsentierenden Zellen und 20 $\mu$g/ml BMA restimuliert und für 40 Tage in Kultur gehalten. Die autologen Lymphoblastoidzellen stammten von einer Zellinie, die durch Transformation der peripheren mononuklearen Blutzellen des Patienten mit 50 % eines Epstein-Barr-Virus enthaltenden Überstands hergestellt wurde, der aus einer 12 Tage-Kultur einer Marmoset-Zellinie (ATCC CRL 1612) und 1 % PHA (Difco Co., Detroit) erhalten wurde.

Anschließend wurden die Zellen gesammelt und in Kulturmedium (RPMI 1640, 2 mM L-Glutamin, 2 mM Na-Pyruvat, 100 U/ml Penicillin, 100 $\mu$g/ml Streptomycin und 10 % fötales Kälberserum (FCS, BRL Gibco, Freiburg)) unter Zusatz von 50 % FCS und 10 % Dimethylsulfoxid eingefroren und in flüssigem Stickstoff aufbewahrt. Aus dieser T-Zellinie wurden T-Zellklone durch Stimulation mit BMA für 8 Tage nach der von van Vliet et al., supra, beschriebenen Methode hergestellt. Die Zellen wurden mit einer Konzentration von 0,5 Zellen pro Loch in Kulturmedium mit 10 % Humanserum in 96 Loch-Flachbodenmikrotiterplatten ausplattiert, die autologe lymphoblastoide Zellen ($4 \times 10^4$ Zellen/Loch, mit 60 Gy bestrahlt), einen Pool peripherer mononuklearer Blutzellen von 6 Donoren ($8 \times 10^4$ Zellen/Loch, mit 40 Gy bestrahlt) und 40 $\mu$g/ml BMA in einem Gesamtvolumen von 200 $\mu$l/Loch enthielten. Nach 2 Tagen wurden 50 $\mu$l frisches Kulturmedium mit 10 % Humanserum und 40 U/ml rekombinantem IL-2 pro Loch zugesetzt. Die wachsenden Klone wurden auf 24 Loch-Kulturplatten (Greiner, Frickenhausen, Deutschland) überführt und wöchentlich durch Zugabe von 10 $\mu$g/ml BMA und bestrahlten autologen Lymphoblastoidzellen restimuliert.

Beispiel 3

Antigen-spezifische Proliferation der T-Zellinie und der T-Zellklone

Nach der Methode von Wank et al. ((1979), Scan. J. Immunol. 9 (6): 499) wurden Proliferationsassays in 96-Loch-Flachbodenplatten durch Inkubation von $10^4$ T-Zellen mit $2,5 \times 10^5$ bestrahltenautologen peripheren mononuklearen Blutzellen und 10 $\mu$g/ml Membranpräparationen verschiedener Quellen für 3 Tage durchgeführt. Als Kontrolle wurden Zellen mit autologen peripheren mononuklearen Blutzellen oder Lymphoblastoidzellen alleine inkubiert. 18 Stunden vor der Beendigung des Tests wurde 1 $\mu$Ci mit Tritium markiertes Methylthymidin ($^3$H-TdR; DuPont de Nemours, Deutschland) zugegeben. Die Proben wurden gesammelt und die Aufnahme von $^3$H-TdR wurde durch Flüssigkeitsszintillationszählung gemessen.

Drei Klone, K2.4, K2.12 und K2.16, die die stärksten Proliferationsreaktionen mit BMA im Vergleich zur Kontrolle zeigten, wurden mit verschiedenen Membranpräparationen getestet. Tabelle 1 faßt die Ergebnisse dieses Experiments zusammen. Die T-Zellinie und die Zellklone zeigten eine starke Proliferation mit Membranantigenpräparationen von Ratteninsulinomzellen (BMA), Ratteninselzellen, humanen Inselzellen und NOD-Mausinselzellen, dagegen praktisch keine Proliferation mit Membranantigenpräparationen von Rattenmilz, humanen SW579 Zellen und CAKI-Zellen oder autologen peripheren mononukleären Blutzellen (PBMC) und Lymphoblastoidzellen.

Tabelle 1: Proliferation einer BMA-spezifischen T-Zellinie und von T-Zellklonen aus einem Typ I Diabetes-Patienten

| Antigene | T-Zellinie | K2.12 | K2.16 | K2.4 |
|---|---|---|---|---|
| Ratteninsulinom-Membranen (BMA) | 81955 ± 7534 | 85726 ± 3745 | 86652 ± 3664 | 87270 ± 2115 |
| Ratteninselzell-membranen | 71346 ± 366 | 85490 ± 3392 | 23586 ± 5612 | 83820 ± 1637 |
| NOD Mausinsel-zellmembranen | 43942 ± 6741 | 91804 ± 4336 | 82448 ± 4103 | 86367 ± 2946 |
| Humane Inselzell-membranen | 92260 ± 3502 | 94533 ± 3409 | 97803 ± 3634 | 58386 ± 2069 |
| Rattenmilz-membranen | 3968 ± 778 | 3074 ± 885 | 4387 ± 846 | 4497 ± 1429 |
| Humane SW579-Membranen | 4910 ± 526 | 6175 ± 469 | 6470 ± 487 | 8642 ± 1771 |
| Humane CAKI-Membranen | 6717 ± 294 | 4953 ± 246 | 6430 ± 114 | 7129 ± 1043 |
| Autologe PBMC | 1619 ± 340 | 863 ± 168 | 1336 ± 263 | 1371 ± 336 |
| Autologe Lympho-plastoidzellen | 7250 ± 10 | 6189 ± 419 | 7988 ± 1745 | 7845 ± 777 |
| - | 2150 ± 212 | 1266 ± 680 | 2343 ± 216 | 1158 ± 868 |

Alle Antigene wurden mit einer Konzentration von 10 $\mu$g/ml verwendet.

Die Proliferationsreaktionen sind als mittlere cpm ± SD von Dreifachkulturen ausgedrückt.

Die mittlere Proliferationsreaktion der T-Zellinie und der Klone, die mit Antigenen allein stimuliert waren, betrug 3250 ± 885.

Versuche zur Erzeugung von BMA-spezifischen T-Zellinien von vier gesunden Donoren waren nicht erfolgreich.

Beispiel 4

T-Zellrezeptor-Repertoireanalyse

Gesamt-RNA aus der T-Zellinie wurde unter Verwendung von RNAzolB (CINNA/BIOTECX, Houston, TX) angereichert. RNA wurde durch AMV-Reverse Transkriptase (Boehringer Mannheim GmbH, Deutschland) unter Verwendung von externen Primern, die spezifisch für die konstanten Regionen des TCR$\alpha$- oder $\beta$-Locus (C$\alpha$1 und C$\beta$1) sind (Broeren et al. (1991), Eur. J. Immunol. 21: 569), in cDNA umgeschrieben. Die cDNA wurde dann unter Verwendung einer Palette von Oligonukleotiden, die aus 18 TCR V$\alpha$ (Davies et al. (1991), N. Engl. J. Med. 325: 238) und 20 V$\beta$-Familien (Choi et al. (1989), Proc. Natl. Acad. Sci. USA 86: 8941; Obata und Kashiwagi (1993), In: HLA 1991, K. Tsuji, M. Aizawa und T. Sasazuki, Hrsg., Oxford Science Publications, Vol 1: 865-916) stammten, in Kombination mit aus der konstanten Region stammenden Primern C$\alpha$2 und C$\beta$2 (Broeren et al. (1991), supra) amplifiziert. Die Bedingungen für die PCR waren wie folgt: Nach einem anfänglichen Denaturierungsschritt bei 94°C für 5 Minuten wurden 30 Zyklen mit drei Schritten bei 94°C, 56°C und 72°C für jeweils 30 Sekunden durchgeführt. Die PCR-Produkte wurden auf 4 % Agarosegelen aufgetrennt und analysiert. Der Prozentanteil von jeder TCR V$\alpha$- oder -$\beta$-Genfamilie wurde durch Division der Bandenintensitiät im Gel durch die Summe der Bandenintensitäten für alle V$\alpha$- oder V$\beta$-Genfamilien ermittelt.

Das TCR V$\alpha$- und V$\beta$-Repertoire der BMA-reaktiven Zellinie wurde nach 8 und 40 Tagen Restimulation mit BMA bestimmt und mit dem von unstimulierten PBMC verglichen. Die Prozentanteile bestimmter TCR V$\alpha$-Gene in Bezug auf die Gesamtzahl von V$\alpha$-Gentranskripten variierte zwischen PBMC und der BMA-stimulierten T-Zellinie zu beiden Zeitpunkten.

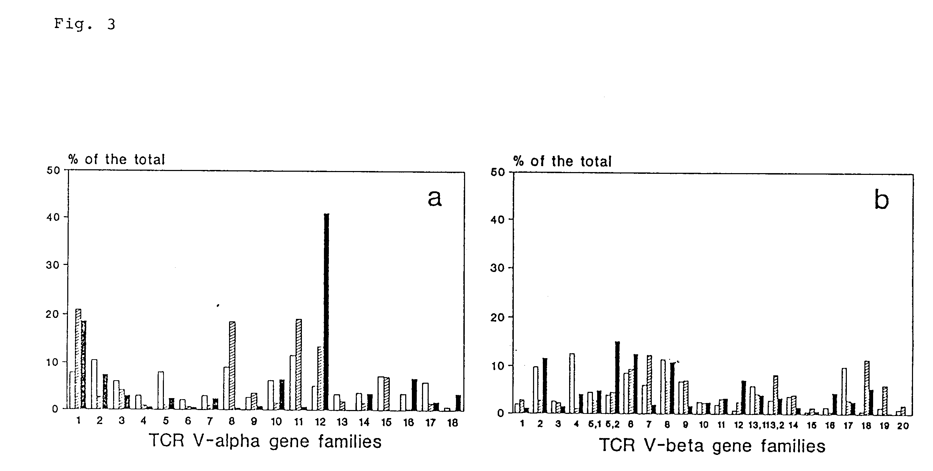

In Figur 3 ist das TCR-Repertoire von unstimulierten PBMC (gepunktete Balken) mit einer 8-tägigen Restimulation (schraffierte Balken) und einer 40-tägigen Restimulation (schwarze Balken) der BMA-spezifischen T-Zellinie verglichen. Nach 8-tägiger Stimulation waren die Anteile an V$\alpha$1, 8, 11 und 12 Genen erhöht, während nach 40-tägiger Stimulation die Expression von V$\alpha$1 und insbesondere V$\alpha$12 stark zunahm.

In Figur 3 ist der Einfluß der BMA-Stimulation auf das V$\beta$-Repertoiregezeigt. Es ist zu erkennen, daß sich die Anteile der V$\beta$5.2, 12, 16 und 18 Gensegmente mit der Zeitdauer der BMA-Stimulierung erhöht.

Beispiel 5

Hemmung der Proliferation durch monoklonale Anti-HLA-Antikörper

Die folgenden monoklonalen Antikörper wurden in den Proliferation-Inhibitionsuntersuchungen verwendet: W6/32 gegen Klasse I-Moleküle (Barnstable et al. (1978), Cell 14:9); L243 gegen HLA-DR (Lampson und Levy (1980), J. Immunol 125: 293); 2aB8 gegen HLA-DR4, 16.23 gegen HLA-DR3 und DRw6 (Johnson et al. (1982), J. Exp. Med. 156: 104), Tü22 gegen HLA-DR und DQ (Ziegler et al. (1986), Immunobiology 171: 77); G2b.2 gegen HLA-DQw1 (Parham et al. (1983), Hum. Immunol 8: 141); LY6 gegen HLA-DQw2 und 7bF4 gegen HLA-DQw3. Die monoklonalen Antikörper 16.23 und G2b.2 wurden den Kulturen als Ascites-Flüssigkeiten mit einer 1/100 Endverdünnung zugesetzt und die restlichen Antikörper wurden als Hybridomüberstände mit einer 1/3 Endverdünnung verwendet.

Die Klone K2.4, K2.12 und K2.16 wurden in Proliferationshemmungsuntersuchungen auf HLA-restringierte Erkennung einer Reaktion gegen BMA unter Verwendung der oben genannten HLA-spezifischen monoklonalen Antikörper getestet.

Das Ergebnis dieses Tests ist in Tabelle 2 dargestellt. Es ist zu erkennen, daß die Proliferationsreaktionen der Klone stark durch den mit HLA-DQ-Molekülen reagierenden monoklonalen Antikörper TÜ22 sowie durch den für HLA-DQw1 spezifischen monoklonalen Antikörper G2b.2 gehemmt wurden. Diese Ergebnisse zeigten, daß die Proliferationsreaktionen aller drei Klone durch DQw1 restringiert wurden.

Somit präsentiert ein HLA-Molekül des Typs DQw1 das Peptid für den T-Zellrezeptor der untersuchten T-Zellklone. Die beiden Allele, die für das DQw1-Molekül kodieren, sind DQA1*0103 und DQB1*0603. Die HLA-DQw1-Nukleotidsequenzen sind bei Marsh und Bodmer (1992), Hum. Immunol. 35: 1 beschrieben. Auf diese Literaturstelle wird ausdrücklich Bezug genommen.

Tabelle 2: Inhibierung der BMA-spezifischen Proliferation durch monoklonale Antikörper

| Monoklonale Antikörper | Spezifität | K2.12 | K2.16 | K2.4 |
|---|---|---|---|---|
| keiner* | - | 93406 ± 2502 | 51003 ± 246 | 54631 ± 5261 |
| W6/32 | Klasse I | 37520 ± 1077 | 38947 ± 1253 | 42091 ± 5096 |
| L243 | HLA-DR | 48685 ± 223 | 35278 ± 1489 | 37252 ± 2076 |
| 2ab8 | HLA-DR4 | 44880 ± 160 | 31112 ± 893 | 32840 ± 2403 |
| 16.23 | HLA-DR3+DRw6 | 32790 ± 298 | 33230 ± 447 | 27850 ± 852 |
| Tü22 | HLA-DQ | 7843 ± 808 | 4149 ± 102 | 5599 ± 644 |
| G2b.2 | HLA-DQw1 | 5652 ± 588 | 1533 ± 107 | 3702 ± 339 |
| LY6 | HLA-DQw2 | 40563 ± 431 | 44485 ± 4620 | 36422 ± 2103 |
| 7bF4 | HLA-DQw3 | 27449 ± 965 | 20503 ± 201 | 23662 ± 2049 |

* Spezifische Proliferation durch Stimulation mit Insulinommembranen (10 μg/ml) ohne Zugabe von monoklonalen Antikörpern.

Die Proliferationsreaktionen sind als mittlere cpm ± SD von Dreifachkulturen ausgedrückt.

EP 0 676 468 A2

Beispiel 6

T-Zellrezeptorsequenzierung

Die Präparation von cDNA aus den T-Zellklonen und die PCR wurden wie oben beschrieben durchgeführt. Die TCR $\alpha$- und $\beta$-Amplifikationsprodukte wurden mit QUIAEX (Diagen, Düsseldorf, Deutschland) aus einem Agarosegel isoliert und nach der von Casanova et al. ((1990), Nucleic Acids Res. 18: 4028) beschriebenen Methode unter Verwendung der internen Sequenzierungsprimer T-C$\alpha$ (Davies et al. (1991), supra) und 3'-C$\beta$ (Choi et al. (1989), supra) und des Enzyms Sequenase 2.0 (USB, Cleveland, Ohio) sequenziert. PCR-Produkte wurden nur mit den Primern V$\alpha$1 und V$\beta$9 für den Klon K2.4, den Primern V$\alpha$5 und V$\beta$8 für den Klon K2.12 und den Primern V$\alpha$12 und V$\beta$16 für den Klon K2.16 erhalten.

Es wurden die Verbindungsregionen der TCR $\alpha$- und $\beta$-Ketten der Klone K2.4, K2.12 und K2.16 einschließlich der J-Gensegmente und der 3'-Region der V-Gensegmente sequenziert. Jeder Klon exprimierte einen unterschiedlichen T-Zellrezeptor (Figur 2). Die TCR $\alpha$-Kette des Klones K2.12 war aus V$\alpha$5.1 und J$\alpha$G, diejenige von Klon K2.16 aus V$\alpha$12.1 und J$\alpha$AB11 und diejenige von Klon K2.4 aus V$\alpha$1.3A und J$\alpha$08-Gensegmenten zusammengesetzt. Die TCR $\beta$-Kette des Klons K2.12 enthielt V$\beta$8.1 und J$\beta$1.5. Diejenige des Klons K2.16 enthielt V$\beta$16.1 und J$\beta$2.3 und diejenige des Klons K2.4 enthielt V$\beta$9.2 und J$\beta$2.4.

In Fig. 1 ist die Nukleinsäuresequenz und die abgeleitete Aminosäuresequenz der TCR $\alpha$- und $\beta$-Verbindungsregionen zwischen V- und J-Gensegmenten der untersuchten Klone gezeigt. Die Bezeichnungen der exprimierten V- und J-Gensegmente wurden gemäß der Nomenklatur des XI International Histocompatibility Workshop (Obata und Kashiwagi (1993), supra) zugeordnet. Grenzen der V- und J-Gensegmente sind angegeben. Die Aminosäuren sind unter Verwendung des Einbuchstabencodes angegeben.

In Fig. 2a ist ein Aminosäuresequenzvergleich der TCR $\alpha$- und $\beta$-CDR1-, CDR2- und CDR3-Sequenzen der Klone K2.12, K2.16 und K2.4 gezeigt. Weiterhin ist in Fig. 2b ein Aminosäuresequenzvergleich der CDR3$\beta$-Sequenzen des Klons K2.12 und des Maus-T-Zellklons 4-1-E.2 (Nakano et al. (1991), J. Exp. Med. 173: 1091) und ein Aminosäuresequenzvergleich der CDR3$\alpha$-Sequenzen des Klons K2.4 und des Maus-T-Zellklons 7-10-D.3 (Nakano et al., supra) dargestellt. Beide Maus-T-Zellklone wurden aus NOD-Mäusen isoliert und induzierten nach Übertragung in gesunde, subletal bestrahlte I-E + NOD Mäuse Insulitis.

SEQUENZPROTOKOLL

SEQ ID NO. 1:
LÄNGE: 15 Nukleotide
     5 Aminosäuren

CTA GAG AAC ACA GGC
 L    E    N    T    G

SEQ ID NO. 2:
LÄNGE: 36 Nukleotide
     12 Aminosäuren

CTG AGT GAG GCC CCG AAT TAT GGT GGT GCT ACA AAC
 L    S    E    A    P    N    Y    G    G    A    T    N

SEQ ID NO. 3:
LÄNGE: 30 Nukleotide
     10 Aminosäuren

GTG ACC ACT CAG TTT TCT GGT GGC TAC AAT
 V    T    T    Q    F    S    G    G    Y    N

SEQ ID NO. 4:
LÄNGE: 27 Nukleotide
     9 Aminosäuren

AGT AGT GAC AGG TTA GGC AAT CAG CCC
 S    S    D    R    L    G    N    Q    P

```
SEQ ID NO. 5:
LÄNGE: 33 Nukleotide
        11 Aminosäuren


AGC CAA GAT CGA CTG AGG GGT GTC GCA GAT ACG
 S   Q   D   R   L   R   G   V   A   D   T



SEQ ID NO. 6:
LÄNGE: 18 Nukleotide
        6 Aminosäuren


AGC CAA GAG GCC GAC ATT
 S   Q   E   A   D   I
```

## Patentansprüche

1.  Nukleinsäure, die für einen Abschnitt aus der CDR3-Region einer Kette eines humanen T-Zellrezeptors codiert, umfassend:
    (a) eine der in SEQ ID NO. 1 - 6 dargestellten Nukleinsäuresequenzen,
    (b) eine Nukleinsäuresequenz, die einer der Sequenzen aus (a) im Rahmen der Degeneration des genetischen Codes entspricht, oder
    (c) eine Nukleinsäuresequenz, die eine Homologie von mindestens 80 % zu einer Nukleinsäuresequenz aus (a) oder/und (b) aufweist.

2.  Nukleinsäure nach Anspruch 1,
    **dadurch gekennzeichnet**,
    daß sie für eine vollständige CDR 3-Region codiert.

3.  Nukleinsäure, die für eine Kette eines humanen T-Zellrezeptors oder eines funktionellen Derivats davon codiert,
    **dadurch gekennzeichnet**,
    daß ihre CDR 3-Region eine Nukleinsäure nach Anspruch 1 oder 2 enthält.

4.  Nukleinsäure nach Anspruch 3,
    **dadurch gekennzeichnet**,
    daß sie für eine α-Kette eines humanen T-Zellrezeptors oder eines funktionellen Derivats davon codiert und ein exprimiertes V-Gensegment aus den Genfamilien Vα1, Vα5 oder Vα12 enthält.

5.  Nukleinsäure nach Anspruch 4,
    **dadurch gekennzeichnet**,
    daß sie ein exprimiertes Vα1.3a-, Vα5.1- oder Vα12.1-Gensegment enthält.

6.  Nukleinsäure nach Anspruch 5,
    **dadurch gekennzeichnet**,
    daß sie ein exprimiertes JαG, JαAB11 oder Jα08-Gensegment enthält.

7. Nukleinsäure nach Anspruch 3,
**dadurch gekennzeichnet**,
daß sie für eine $\beta$-Kette eines humanen T-zellrezeptors oder eines funktionellen Derivats davon codiert und ein exprimiertes V-Gensegment aus den Genfamilien V$\beta$8, V$\beta$9 oder V$\beta$16 enthält.

8. Nukleinsäure nach Anspruch 7,
**dadurch gekennzeichnet**,
daß sie ein exprimiertes V$\beta$8.1-, V$\beta$9.2- oder V$\beta$16.1-Gensegment enthält.

9. Nukleinsäure nach Anspruch 7 oder 8,
**dadurch gekennzeichnet**,
daß sie ein exprimiertes J$\beta$1.5-, J$\beta$2.4- oder J$\beta$2.3-Gensegment enthält.

10. Vektor,
**dadurch gekennzeichnet**,
daß er mindestens eine Kopie einer Nukleinsäure nach einem der Ansprüche 1 bis 9 enthält.

11. Zelle,
**dadurch gekennzeichnet**,
daß sie mit einer Nukleinsäure nach einem der Ansprüche 1 bis 9 oder einem Vektor nach Anspruch 10 transformiert ist.

12. Polypeptid,
**dadurch gekennzeichnet**,
daß es von einer Nukleinsäure nach einem der Ansprüche 1 bis 9 codiert ist.

13. Polypeptid nach Anspruch 12,
**dadurch gekennzeichnet**,
daß es eine Kette eines humanen T-Zellrezeptors oder ein funktionelles Derivat davon ist.

14. Polypeptid, das eine Kette eines humanen T-Zellrezeptors oder ein funktionelles Derivat davon ist und als Abschnitt der CDR3-Region
(a) eine Aminosäuresequenz mit einer Homologie von mindestens 80 % zu den in SEQ ID NO. 1 - 6 dargestellten Aminosäuresequenzen, oder
(b) eine Aminosäuresequenz mit einer äquivalenten Erkennungsspezifität für die Peptidkomponente des T-Zellrezeptorliganden aufweist.

15. Polypeptid nach Anspruch 13 oder 14,
**dadurch gekennzeichnet**,
daß es als Abschnitt der CDR3-Region eine der in SEQ ID NO. 1 - 6 dargestellten Aminosäuresequenzen aufweist.

16. Polypeptid nach Anspruch 13 oder 14,
**dadurch gekennzeichnet**,
daß es als Abschnitt der CDR3-Region eine aus den in SEQ ID NO. 1 - 6 dargestellten Aminosäuresequenzen durch einen oder mehrere Aminosäureaustausche abgeleitete Sequenz aufweist.

17. Polypeptid nach Anspruch 16,
**dadurch gekennzeichnet**,
daß es als Abschnitt der CDR3-Region eine aus den in SEQ ID NO. 1 - 6 gezeigten Aminosäuresequenzen durch einen oder mehrere konservative Aminosäureaustausche abgeleitete Aminosäuresequenzen aufweist.

18. Polypeptid,
**dadurch gekennzeichnet**,
daß es T-Zellrezeptor-Eigenschaften aufweist und aus zwei Polypeptiden nach einem der Ansprüche 12 bis 17 als Untereinheiten aufgebaut ist, wobei eine Polypeptiduntereinheit eine $\alpha$-Kette eines humanen T-Zellrezeptors oder ein funktionelles Derivat davon ist und die andere Polypeptiduntereinheit eine $\beta$-

Kette eines humanen T-Zellrezeptors oder ein funktionelles Derivat davon ist.

19. Antikörper gegen ein Polypeptid nach einem der Ansprüche 12 bis 18, der gegen eine für die Erkennung des Peptidliganden verantwortliche Region gerichtet ist.

20. Antikörper nach Anspruch 19,
**dadurch gekennzeichnet**,
daß er gegen eine CDR3-Region gerichtet ist.

21. T-Zelle,
**dadurch gekennzeichnet**,
daß sie einen T-Zellrezeptor nach Anspruch 18 enthält.

22. Pharmazeutische Zusammensetzung, die als aktive Komponente eine Nukleinsäure nach einem der Ansprüche 1 bis 9, ein Polypeptid nach einem der Ansprüche 12 bis 18, einen Antikörper nach Anspruch 19 oder 20 oder eine T-Zelle nach Anspruch 21 enthält.

23. Verwendung einer pharmazeutischen Zusammensetzung nach Anspruch 21 zur Herstellung eines diagnostischen oder/und therapeutischen Mittels für Typ I-Diabetes.

24. Verwendung eines Antikörpers nach Anspruch 19 oder 20 zur Herstellung eines Impfstoffes gegen autoreaktive T-Zellpopulationen.

25. Verwendung eines Polypeptids nach einem der Ansprüche 12 bis 18 oder einer T-Zelle nach Anspruch 21 zur Herstellung eines Immunogens.

26. Verwendung nach Anspruch 25,
**dadurch gekennzeichnet**,
daß die T-Zelle attenuiert wird.

27. Verwendung nach Anspruch 26,
**dadurch gekennzeichnet**,
daß die Attenuierung der T-Zelle durch Bestrahlung oder/und Hitzebehandlung erfolgt.

28. Komplex, umfassend ein Polypeptid nach Anspruch 18 und ein Peptid-präsentierendes HLA-Molekül der Klasse DQw1.

Figur 1

Jα

| Clon. | Vα | N | Jα |
|---|---|---|---|

**K2.12**

Vα:
```
 C   A   L
TGT GCT CTA G
Vα5.1 (IGRa10)
```
N:
```
 E
AG
```
Jα:
```
 N   T   G   K   L   I   F
AAC ACA GGC AAA CTA ATC TTT
           JαG
```

**K2.16**

Vα:
```
 C   A   L   S   E
TGT GCT CTG AGT GAG GC
Vα 12.1 (PGA5)
```
N:
```
   A   P   N   Y
C CCG AAT TA
```
Jα:
```
   G   A   T   N   K   L   I   F
T GGT GCT ACA AAC AAG CTC ATC TTT
            JαAB11
```

**K2.4**

Vα:
```
 C   A   V
TGT GCT GTG A
Vα1.3a (11wT7)
```
N:
```
    T   T   Q   F   S
CC ACT CAG TTT T
```
Jα:
```
    G   Y   N   K   L   I   F
CT GGT GGC TAC AAT AAG CTG ATT TTT
           Jα08
```

---

| Clon. | Vβ | NDN | Jβ |
|---|---|---|---|

**K2.12**

Vβ:
```
 C   A   S   S
TGT GCC AGC AGT
Vβ8.1 (YT35)
```
NDN:
```
 S   D   R   L   G
AGT GAC AGG TTA G
```
Jβ:
```
   N   Q   P   Q   H   F
GC AAT CAG CCC CAG CAT TTT
          Jβ 1.5
```

**K2.16**

Vβ:
```
 C   A   S   S   Q
TGT GCC AGC AGC CAA GA
Vβ16.1(HBVP42)
```
NDN:
```
  D   R   L   R   G   V   A
T CGA CTG AGG GGT GTC G
```
Jβ:
```
   D   T   Q   Y   F
CA GAT ACG CAG TAT TTT
          Jβ 2.3
```

**K2.4**

Vβ:
```
 C   A   S   S   Q
TGT GCC AGC AGC CAA G
Vβ9.2 (IGRb20)
```
NDN:
```
 E   A   D
AG GCC G
```
Jβ:
```
   I   Q   Y   F
AC ATT CAG TAC TTC
          Jβ 2.4
```

Fig. 2

**a**

| CLONE | Vα | C D R 1 | | | | | | | C D R 2 | | | | | | | | | | | |
|---|---|---|---|---|---|---|---|---|---|---|---|---|---|---|---|---|---|---|---|---|---|
| K2.12 | 5.1 | T | N | Y | S | P | A | Y | L | I | R | E | N | E | K | E | K | R | K | E |
| K2.16 | 12.1 | E | T | R | D | T | T | Y | L | I | R | R | N | S | F | D | E | Q | N | E |
| K2.4 | 1.3a | S | S | S | V | S | V | Y | L | L | K | Y | L | S | G | S | T | L | V | E |

| | C D R 3 | | | | | | | | | | | | | | | | | | Jα |
|---|---|---|---|---|---|---|---|---|---|---|---|---|---|---|---|---|---|---|---|---|
| | C | A | L | | E | | | | | N | T | G | K | L | I | F | G | | G |
| | C | A | L | S | E | A | P | N | Y | G | G | A | T | N | K | L | I | F G | AB11 |
| | C | A | V | T | T | | | Q | F | S | G | G | Y | N | K | L | I | F G | 08 |

| CLONE | Vβ | C D R 1 | | | | | | | C D R 2 | | | | | | | | | | | |
|---|---|---|---|---|---|---|---|---|---|---|---|---|---|---|---|---|---|---|---|---|---|
| K2.12 | 8.1 | P | I | S | G | H | N | S | Y | F | N | N | N | V | P | I | D | D | S | G M P |
| K2.16 | 16.1 | P | I | S | G | H | D | N | H | F | V | K | E | S | K | Q | D | E | S | G M P |
| K2.4 | 9.2 | Q | N | L | G | H | D | T | S | Y | N | N | K | E | L | I | I | N | E | T V P |

| | C D R 3 | | | | | | | | | | | | | | | | | Jβ |
|---|---|---|---|---|---|---|---|---|---|---|---|---|---|---|---|---|---|---|---|
| | C | A | S | S | S | D | R | L | | | G | N | Q | P | Q | H | F G | 1.5 |
| | C | A | S | S | Q | D | R | L | R | G | V | A | D | T | Q | Y | F G | 2.3 |
| | C | A | S | S | Q | E | | | | | | A | D | I | Q | Y | F G | 2.4 |

**b**

| CLONE | Vβ | C D R 3 | | | | | | | | | | | | | | | Jβ |
|---|---|---|---|---|---|---|---|---|---|---|---|---|---|---|---|---|---|---|
| K2.12 | h8.1 | C | A | S | S | S | D | R | L | G | N | Q | | P | Q | H | F G | 1.5 |
| 4-1-E.2 | m12 | C | A | S | | | | R | L | G | N | Q | D | T | Q | Y | F G | 2.5 |

| CLONE | Vα | C D R 3 | | | | | | | | | | | | | | | Jα |
|---|---|---|---|---|---|---|---|---|---|---|---|---|---|---|---|---|---|---|
| K2.4 | h1.3a | C | A | V | T | T | Q | F | S | G | G | Y | N | K | L | I | F G | 08 |
| 7-10-D.3 | m1.1 | C | A | V | | | | | S | P | G | Y | N | K | L | T | F G | D3 |

16

Fig. 3